# EUROPEAN PATENT APPLICATION

(11) **EP 1 783 128 A1**
(43) Date of publication of application: **09.05.2007**
(21) Application number: 05024187.6
(22) Date of filing: 07.11.2005
(51) Int. Cl.: C07D 471/04, C07D 519/00, A61P 31/00, A61K 31/437, A01N 43/90

(54) **Heterocyclic compounds and their use as antifouling agents**

(71) Applicant: Eidgenössische Technische Hochschule Zürich, 8092 Zürich (CH); Universität Zürich, 8006 Zürich (CH)
(72) Inventor: Blom, Judith, CH-8057 Zürich (CH); Gademann, Karl, CH-8604 Volketswil (CH)
(74) Representative: Schaad, Balass, Menzl & Partner AG

(57) **Abstract**

The present invention relates to a nostocarboline compound of the formula (I) and its use as a biocidal agent.

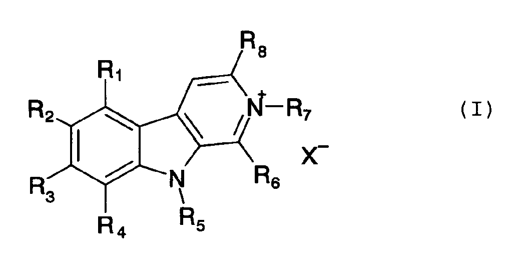

In this formula R₁, R₃, R₄ and R₆ are selected from the group consisting of H, F, Cl, Br, I, a C₁-C₂₀ aliphatic residue, and a C₁-C₂₀ aliphatic residue comprising a carbocyclic or heterocyclic residue;
R₂ is selected from the group consisting of H and Cl;
R₅ is selected from the group consisting of H, a C₁-C₂₀ aliphatic residue, and a C₁-C₂₀ aliphatic residue comprising a carbocyclic or heterocyclic residue;
R₇ is selected from the group consisting of a C₁-C₂₀ aliphatic residue, a C₁-C₂₀ aliphatic residue comprising a carbocyclic or heterocyclic residue, and a linker bound to a bioactive factor;
R₈ is selected from the group consisting of H, a C₁-C₆ aliphatic carboxylic acid, ester, or amide, and a C₃-C₂₀ carbocyclic residue; and
X⁻ is a negatively charged ion.

## Description

The present invention relates to antifouling agents, which are useful to prevent the formation of a biofilm on water exposed surfaces, their uses in various environments and a method of producing these agents.

Microorganisms occur either naturally or as a consequence of pollution in almost any environment where water is present. Interaction of microorganisms with artificial surfaces eventually leads to a process known as biofouling. Briefly, biofouling is an undesirable accumulation of microorganisms such as bacteria, cyanobacteria, algae etc. on submerged structures, e.g. ship's hulls.

Biofilm accumulation can achieve considerable masses which, again referring to ships as an example, negatively affects their manouverability or carrying capacity. Further, the biofouling leads to the destruction of the affected surface. Of course, also other structures are affected such as oil platforms, seawater pipelines etc. Repairing and preventing such damages incurs annual costs of several billion Swiss Francs.

Other fields where biofouling causes problems are for instance industrial waters, swimming pools, cooling systems and the like. The sanitation of swimming pools relates to a field which imposes particularly high requirements on sanitation or antifouling agents in terms of their efficiency and selectivity since man is directly exposed to the agents employed.

Antifouling agents are usually used in the form of a protective coat or paint which is applied on the surface exposed to fouling. In closed systems the antifouling agent may either be applied on the submerged surfaces or it may be directly added to the water.

In the past various heavy metals have been used to achieve paints having antifouling and also anticorrosive properties. Copper has a long tradition as a antifouling additive in paints. Normally Cu₂O is used as biocidal agent, but also copper metal and various other copper compounds were employed. A problem associated with copper based antifouling agents is an increasing resistance of microorganisms to such agents.

Arsenic and mercury were also used as biocides in antifouling paints. Both, inorganic mercury and organomercury has been used.

Further, organolead and inorganic lead compounds have been employed in antifouling coats.

At the end of the 60's a new generation of antifouling agents, organotin compounds, replaced the traditional copper-based antifouling agents. The main reason was the excellent antifouling property, organotin compounds showed. There are basically two different types of protective coats or paints that are used. In the first type, the organotin compound is freely dispersed in the paint. Once applied the organotin leaches out of the paint matrix based on diffusion. Typically, such protective coats or paints show a high initial and uncontrolled burst of the active agent. Subsequently, the release decreases until the antifouling properties of the paint are exhausted. A second type of protective paint employed is referred to as tributyltin (TBT) self polishing copolymer (SPC). Nowadays, this type is widely used. Copolymer systems are based on a combination of biologically active resins and other active compounds, typically TBT copolymer resins. The TBT biocide is covalently bound within a copolymer resin system. The coating on a ship that has been painted with a TBT-SPC paint system will react, by hydrolysis with sea water, resulting in the slow release of TBT which prevents biofouling. The remaining surface of the paint system is eroded by moving seawater due to its mechanical weakness, resulting in the exposure of a fresh surface of TBT polymer.

The antifouling agents described above show several disadvantages which render them more and more useless or impair at least their efficiency.

For instance, in the last years copper based antifouling agents became less efficient because of an increasing resistance of the target organisms against such agents. Thus, to achieve the same effect one has to use higher amounts which in turn increases unwanted toxicity for other organisms.

Once it became clear that TBT compounds may adhere to silt, these agents came under scrutiny by environmental groups and eventually into the focus of environmental legislation. The use of TBT based biocides was subsequently either banned or at least restricted. Clearly, also copper, lead or mercury based antifouling agents contributed to some of the marine pollution in coastal waters found today. Low (bio-)degradability increases the tendency of these compounds to accumulate in the environment.

A further problem is the non-existing selectivity displayed by most of the employed antifouling agents. This means microorganisms are unspecifically killed or blocked in their growth independent from their individual contribution to the fouling problem. In such cases the only way to achieve at least some "selectivity" may be managed through the effective concentration used. However this requires great differences in susceptibility to the antifouling compound between different organisms.

WO 2004/101500 describes an algicidal compound eventually releasing cyanide as active agent.

The problem of the present invention was to provide antifouling agents which are effective in low amounts, show an excellent selectivity between different microorganisms, show a good stability without being virtually non-(bio-)degradable. In addition, they have to be produced in simple and cheap manner.

The problem is solved by a compound according to claim 1. Further preferred embodiments are the subject-matter of the dependent claims.

It has been shown that nostocarboline compounds according to formula (I) as described below are effective in very low amounts. Further, said compounds have an excellent selectivity displaying biocidal effect against photosynthetic microorganisms. Nostocarboline compounds according to the present invention have a good stability but despite their stability do not accumulate in their environment and they can be produced in very cost-effective manner.

Nostocarboline compounds according to the present invention are used as biocidal agents. The nostocarboline compounds have a general structure according to formula (I) 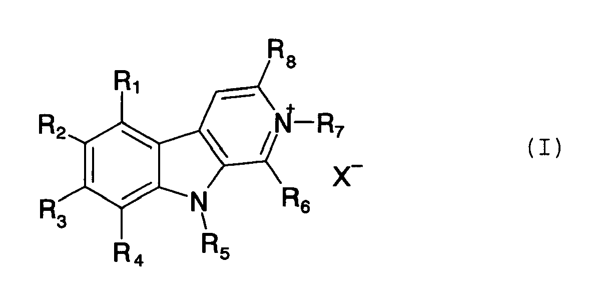 wherein
R₁, R₃, R₄ and R₆ are independently selected from the group consisting of H, F, Cl, Br, I, a saturated or unsaturated, substituted or unsubstituted C₁-C₂₀ aliphatic residue with straight or branched chain optionally having one or more heteroatom at the beginning or in the chain, and a saturated or unsaturated, substituted or unsubstituted C₁-C₂₀ aliphatic residue with straight or branched chain comprising a carbocyclic or heterocyclic residue;
R₂ is selected from the group consisting of H and Cl;
R₅ is selected from the group consisting of H, a saturated or unsaturated, substituted or unsubstituted C₁-C₂₀ aliphatic residue with straight or branched chain optionally having one or more heteroatom in the chain, and a saturated or unsaturated, substituted or unsubstituted C₁-C₂₀ aliphatic residue with straight or branched chain comprising a carbocyclic or heterocyclic residue;
R₇ is selected from the group consisting of a saturated or unsaturated, substituted or unsubstituted C₁-C₂₀ aliphatic residue with straight or branched chain optionally having one or more heteroatom in the chain, or a saturated or unsaturated, substituted or unsubstituted C₁-C₂₀ aliphatic residue with straight or branched chain comprising a carbocyclic or heterocyclic residue or a linker bound to a bioactive factor;
R₈ is selected from the group consisting of H, saturated or unsaturated, substituted or unsubstituted C₁-C₆ aliphatic carboxylic acid, ester, amide with straight or branched chain, and a C₃-C₂₀ carbocyclic residue; and
X⁻ is an negatively charged ion.
As used herein "a saturated or unsaturated, substituted or unsubstitued C₁ to C₂₀ aliphatic residue with straight or branched chain" is intended to mean alkyl chains with 1 to 20 carbon atoms, which may be straight or branched, substituted or unsubstituted and saturated or unsaturated. Examples are methyl, ethyl, n-propyl, i-propyl, n-butyl, i-butyl, sec-butyl, t-butyl, pentyl, hexyl, heptyl, octyl, nonyl, decyl, undecyl, dodecyl, tridecyl, ethenyl, 1-propenyl, 2-propenyl, 1-butenyl, 2-butenyl, 3-butenyl, 3-methyl-2-butenyl, 2-pentenyl, 3-pentenyl, hexenyl, ethynyl, 1-propynyl, 2-propynyl, 1-butynyl, 2-butynyl, 3-butynyl, allyl and the like. Preferred are methyl, ethyl, n-propyl, i-propyl and n-butyl. Most preferred are methyl and ethyl. Said aliphatic residue may be substituted. The term substituted is intended to mean the replacement of a H of the aliphatic residue by a halogen, O, N or S. Examples for substituted aliphatic residues are aminoethyl, aminopropyl, amino-i-propyl, aminobutyl, amino-i-butyl, amino-s-butyl, amino-t-butyl, hydroxyethyl, hydroxypropyl, hydroxy-i-propyl, hydroxylbutyl, hydroxy-i-butyl, hydroxy -s-butyl, hydroxy -t-butyl. Preferred are aminoethyl, aminopropyl, hydroxyethyl and hydroxybutyl. Most preferred are aminoethyl and hydroxyethyl.

Said aliphatic residue may optionally have one or more heteroatom selected from the group consisting of N, O, and S at the beginning or in the chain. If the heteroatom is at the beginning of the chain, the heteroatom is bound directly to the ring system. Examples for aliphatic residues having a heteroatom at the beginning of the chain are methoxy, ethoxy, n-propoxy, i-propoxy, n-butoxy, s-butoxy, t-butoxy, n-pentoxy, and s-pentoxy. Preferred are methoxy, ethoxy, n-propoxy, i-propoxy, n-butoxy, s-butoxy, t-butoxy. Most preferred are methoxy and ethoxy.

If the optional heteroatom of the saturated or unsaturated, substituted or unsubstituted aliphatic residue with the straight or branched chain is in the chain, the heteroatom may be bound for example as ether, thioether or imine.

As used herein, carbocyclic residue is intended to mean any stable 3- to 7-membered monocyclic or bicyclic or 7-to 20-membered bicyclic or tricyclic, any of which may be saturated, partially unsaturated, or aromatic. Examples of such carbocycles include, but are not limited to, cyclopropyl, cyclobutyl, cyclopentyl, cyclohexyl, cycloheptyl, adamantyl, cyclooctyl, [3.3.0]bicyclooctane, [4.3.0]bicyclononane, [4.4.0]bicyclodecane (decalin), [2.2.2]bicyclooctane, fluorenyl, phenyl, benzyl, naphthyl, indanyl, adamantyl, indenyl, antracenyl or tetrahydronaphthyl (tetralin). Preferred "carbocycle" are cyclopropyl, cyclobutyl, cyclopentyl, cyclohexyl, phenyl, naphtyl and antracenyl. Most preferred are cyclopentyl, cyclohexyl, naphtyl and antracenyl. The carbocyclic residue may be bound directly to the ring system or bound to an alkyl residue, for example a methyl or ethyl residue. Preferably the carbocylic residue is directly bound to the ring system for R₁ to R₄ and R₆ and preferably bound to methyl for R₅ and R₇.

As used herein, the term heterocyclic residue is intended to mean a stable 5- to 7-membered monocyclic or bicyclic or 7- to 14-membered bicyclic heterocyclic ring which is saturated partially unsaturated or unsaturated (aromatic), and which consists of carbon atoms and 1, 2, 3 or 4 heteroatoms independently selected from the group consisting of N, O and S and including any bicyclic group in which any of the above-defined heterocyclic rings is fused to a benzene ring.

Examples of heterocycles include, but are not limited to, 1H-indazole, 2-pyrrolidonyl, 2H,6H-1,5,2-dithiazinyl, 2H-pyrrolyl, 3H-indolyl, 4-piperidonyl, 4aH-carbazole, 4H-quinolizinyl, 6H-1,2,5-thiadiazinyl, acridinyl, azocinyl, benzimidazolyl, benzofuranyl, benzothiofuranyl, benzothiophenyl, benzoxazolyl, benzthiazolyl, benztriazolyl, benztetrazolyl, benzisoxazolyl, benzisothiazolyl, benzimidazalonyl, carbazolyl, 4aH-carbazolyl, chromanyl, chromenyl, cinnolinyl, decahydroquinolinyl, 2H,6H-1,5,2-dithiazinyl, dihydrofuro[2,3-b]tetrahydrofuran, furanyl, furazanyl, imidazolidinyl, imidazolinyl, imidazolyl, 1H-indazolyl, indolenyl, indolinyl, indolizinyl, indolyl, isobenzofuranyl, isochromanyl, isoindazolyl, isoindolinyl, isoindolyl, isoquinolinyl, isothiazolyl, isoxazolyl, morpholinyl, naphthyridinyl, octahydroisoquinolinyl, oxadiazolyl, 1,2,3-oxadiazolyl, 1,2,4-oxadiazolyl, 1,2,5-oxadiazolyl, 1,3,4-oxadiazolyl, oxazolidinyl, oxazolyl, oxazolidinylperimidinyl, phenanthridinyl, phenanthrolinyl, phenarsazinyl, phenazinyl, phenothiazinyl, phenoxathiinyl, phenoxazinyl, phthalazinyl, piperazinyl, piperidinyl, pteridinyl, piperidonyl, 4-piperidonyl, pteridinyl, purinyl, pyranyl, pyrazinyl, pyrazolidinyl, pyrazolinyl, pyrazolyl, pyridazinyl, pyridooxazole, pyridoimidazole, pyridothiazole, pyridinyl, pyridyl, pyrimidinyl, pyrrolidinyl, pyrrolinyl, pyrrolyl, quinazolinyl, quinolinyl, 4H-quinolizinyl, quinoxalinyl, quinuclidinyl, carbolinyl, tetrahydrofuranyl, tetrahydroisoquinolinyl, tetrahydroquinolinyl, 6H-1,2,5-thiadiazinyl, 1,2,3-thiadiazolyl, 1,2,4-thiadiazolyl, 1,2,5-thiadiazolyl, 1,3,4-thiadiazolyl, thianthrenyl, thiazolyl, thienyl, thienothiazolyl, thienooxazolyl, thienoimidazolyl, thiophenyl, triazinyl, 1,2,3-triazolyl, 1,2,4-triazolyl, 1,2,5-triazolyl, 1,3,4-triazolyl, xanthenyl. Preferred 5 to 10 membered heterocycles include, but are not limited to, pyridinyl, pyrimidinyl, triazinyl, furanyl, thienyl, thiazolyl, pyrrolyl, pyrazolyl, imidazolyl, oxazolyl, isoxazolyl, tetrazolyl, benzofuranyl, benzothiofuranyl, indolyl, benzimidazolyl, 1H-indazolyl, oxazolidinyl, isoxazolidinyl, benzotriazolyl, benzisoxazolyl, oxindolyl, benzoxazolinyl, quinolinyl, and isoquinolinyl. Preferred 5 to 6 membered heterocycles include, but are not limited to, pyridinyl, pyrimidinyl, triazinyl, furanyl, thienyl, thiazolyl, pyrrolyl, piperazinyl, piperidinyl, pyrazolyl, imidazolyl, oxazolyl, isoxazolyl, tetrazolyl; more preferred 5 to 6 membered heterocycles include, but are not limited to, pyridinyl, pyrimidinyl, triazinyl, furanyl, thienyl, thiazolyl, piperazinyl, piperidinyl, pyrazolyl, imidazolyl, and tetrazolyl.

The heterocyclic residue may be bound directly to the ring system or bound to an alkyl residue, for example a methyl or ethyl residue. Preferably the heterocylic residue is directly bound to the ring system for R₁ to R₄ and R₆ and preferably bound to methyl for R₅ and R₇.

The definition of the term "a saturated or unsaturated, substituted or unsubstitued C₁ to C₂₀ aliphatic residue with straight or branched chain optionally having one or more heteroatom in the chain", as mentioned for the residues R₅ and R₇ has the same meaning as for the residues R₁ to R₄ and R₆ beside the fact, that the optional heteroatom must not be at the beginning of the chain, meaning that no heteroatom is bound the nitrogen present in the ring system.

As used herein "a C₁ to C₆ carboxylic acid" is intended to mean alkyl chains with 1 to 6 carbon atoms, which may be straight or branched, substituted or unsubstituted and saturated or unsaturated either comprising a carboxy, a ester or amide residue. Examples are acetic acid, ethyl carboxylic acid, propyl carboxylic acid, butyl carboxylic acid, pentyl carboxylic acid. This applies analogously to carboxylic esters and carboxylic amides.

R₁, R₃, R₄ and R₆ are preferably selected from the group consisting of H, F, Cl, Br, I and unsubstituted, saturated or unsaturated C₁ to C₅ aliphatic residues. R₅ is preferably selected from the group consisting of H and unsubstituted, saturated or unsaturated C₁ to C₅ residues.

R₂ is selected from the group consisting of H and Cl.

R₇ is preferably selected from the group consisting unsubstituted, saturated or unsaturated C₁ to C₅ residues, phenyl, benzyl, naphtyl and anthranyl residues. R₇ may also be a nostocarboline compound. In such a case the second nostocarboline compound is bound to the first nostocarboline compound via a linker as defined below. This results in a nostocarboline dimer.

R₁, R₃, R₄ and R₆ are more preferably selected from the group consisting of H and Cl. R₅ is more preferably selected from the group consisting of unsubstituted, saturated or unsaturated C₁ to C₅ residues. R₆ is more preferably selected from the group consisting unsubstituted, saturated or unsaturated C₁ to C₅ residues and benzyl.

X⁻ is a negatively charged ion and is preferably selected from the group consisting of F, Cl, Br, I,CF₃CO₂, PO₄, HPO₄, H₂PO₄, SO₄, HSO₄, NO₃, OH, citrate, fumarate, tartrate, aspartate and glutamate.

In a preferred embodiment of the present invention R₂ is Cl. Said nostocarboline compounds show excellent results with respect to their biocidal activity, selectivity and the very low environmental burden they cause. In addition, these compounds can be produced in a cost-effective manner.

Often, it is not possible that a bioactive factor is directly bound to another compound, e.g. due to sterical reasons. Therefore, bioactive factors are usually bound to other compounds via a linker as further defined below.

As used herein, the term "linker" is intended to mean a is intended to mean alkyl chains with 1 to 20 carbon atoms, which may be straight or branched and saturated or unsaturated. Examples are methylen, ethylen, n-propylen, i-propylen, n-butylen, i-butylen, sec-butylen, t-butylen, pentylen, hexylen, heptylen, octylen, nonylen, decylen, undecylen, dodecylen, tridecylen, ethenylen, 1-propenylen, 2-propenylen, 1-butenylen, 2-butenylen, 3-butenylen, 3-methyl-2-butenylen, 2-pentenylen, 3-pentenylen, hexenylen, ethynylen, 1-propynylen, 2-propynylen, 1-butynylen, 2-butynylen, 3-butynylen, and the like. Preferred are methylen, ethylen, n-propylen, i-propylen, n-butylen and dimethylbenzene. Most preferred are methylen, ethylen, and dimethylbenzene. The linker may also comprise one or more heteroatom in the chain or a carbocycle or heterocycle. The heteroatom must not be at the beginning of the chain or at the end of the chain in the case of dimer, meaning that no heteroatom is bound the nitrogen present in the ring system. The heteroatom may be an ether or thioether linkage. Heteroatoms in the chain may also be part of ester, amide or urethane linkage. Depending on the type of the linkage the linker can comprise hydrolytically unstable bonds, thus accounting to some extent for the degradation of the compounds.

The term bioactive factor is intended to mean to mean any compound exerting an inhibitory effect on microorganisms. Examples are nostocarbolines and antibiotics, e.g piperacillin, mezlocillin, ampicillin, amoxicillin, imipenem, meropenem, ertapenem, cephalosporines, flucloxacillin, methicillin, oxacillin, aztreonam, penicillin G, penicillin V, clavulanic acid, sulbactam, tazobactam, sultacillin, fosfomycin, teicoplanin, vancomycin, bacitracin, colistin, tyorthricin, amikacin, gentamycin, netilmycin, tobramycin, chloramphenicol, ciprofloxacin, fosfomycin, vancomycin, linezolid and the like.

The bioactive factor is preferably selected from the group consisting of ciprofloxacin (as shown below in formula (XIII)), fosfomycin, vancomycin linezolid and a nostocarboline compound (forming a dimer; an example is shown in formula XIV). Most preferably the bioactive factor is selected from the group consisting of ciprofloxacin, fosfomycin, vancomycin and linezolid.

It has been shown that the nostocarboline compounds according to the present invention can be linked to other bioactive factors. Said bioactive factor can be a nostocarboline compound according to the present invention. This yields a dimer comprising two nostocarboline compounds as shown for example in formula (XIV) below.

The excellent biocidal activity of nostocarboline compounds according to the present invention can be used in antifouling agents or herbicides. Antifouling agents are often used in the form of protective coats or paints comprising at least one antifouling agent.

Nostocarboline compounds according to the present invention are particularly suited to be employed in protective coats for surfaces submerged in water, e.g ship's hulls, boats, oil platforms etc. Such surfaces are especially exposed to biofouling activities and need to be treated with protective coats having very good antifouling properties.

Nostocarboline compounds having excellent biocidal properties, very good stability and which are harmless for the environment have a structure according to formula (I) wherein R₂ is Cl (as shown in formula (I) and (II))

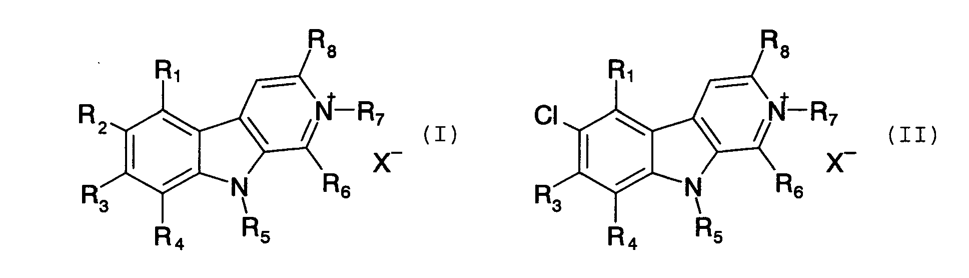

In a preferred embodiment of the present invention R₁, R₃, R₄ and R₆ are preferably selected from the group consisting of H, F, Cl, Br, I and unsubstituted, saturated or unsaturated C₁ to C₅ aliphatic residues. R₅ is preferably selected from the group consisting of H and unsubstituted, saturated or unsaturated C₁ to C₅ residues.

R₂ is Cl.

R₇ is preferably selected from the group consisting unsubstituted, saturated or unsaturated C₁ to C₅ residues, phenyl, benzyl, naphtyl and anthranyl residues. R₇ may also be a nostocarboline compound. In such a case the second nostocarboline compound is bound to the first nostocarboline compound via a linker as defined below. This results in a nostocarboline dimer.

R₁, R₃, R₄ and R₆ are more preferably selected from the group consisting of H and Cl. R₅ is more preferably selected from the group consisting of unsubstituted, saturated or unsaturated C₁ to C₅ residues. R₆ is more preferably selected from the group consisting unsubstituted, saturated or unsaturated C₁ to C₅ residues and benzyl.

X⁻ is a negatively charged ion and is preferably selected from the group consisting of F, Cl, Br, I,CF₃CO₂, PO₄, HPO₄, H₂PO₄, SO₄, HSO₄, NO₃, OH, citrate, fumarate, tartrate, aspartate and glutamate.

Said nostocarboline compounds show very good biocidal activity and good selectivity.

In another preferred embodiment R₇ is more preferably selected from the group consisting unsubstituted, saturated or unsaturated C₂ to C₅ residues, phenyl, benzyl, naphtyl and anthranyl residues. R₇ may also be a nostocarboline compound. In such a case the second nostocarboline compound is bound to the first nostocarboline compound via a linker as defined below. This results in a nostocarboline dimer.

Said nostocarboline compounds show very good biocidal activity and good selectivity.

Further preferred embodiments are described below. Good results with respect to biocidal activity are achieved using nostocarboline compounds having the general formula (I) wherein the residues R₁ and R₃ are H. The structure of said nostocarboline compounds is shown in formula (III)

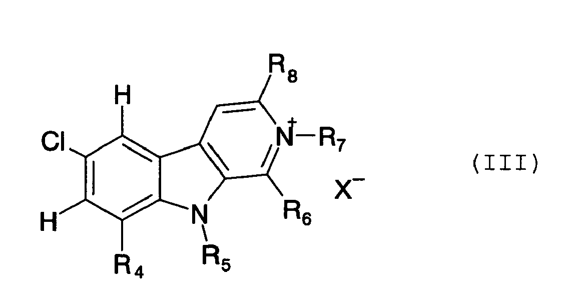

A further preferred embodiment relates to nostocarboline compounds according to the general formula (I) wherein R₂ and R₄ are Cl (structure shown in formula (IV)). Said compounds show good antifouling properties and can be produced in a cheap and reliable process.

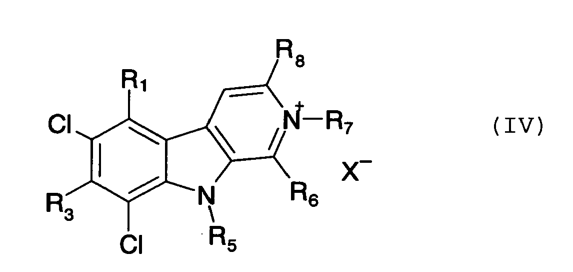

In another preferred embodiment the nostocarboline compound carries substituents R₂ and R₄ wherein either R₂ is Cl and R₄ is H or vice versa. The respective structures are shown in the formulas (V) and (VI). These nostocarboline compounds show potent antifouling properties.

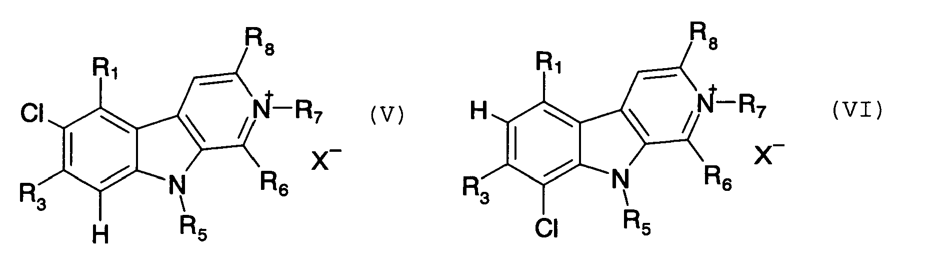

Further preferred embodiments relate to the following specific nostocarboline compounds which all show very good biocidal activity, excellent selectivity in exerting their biocical effect. The structure of said nostocarboline compounds is shown in formulas (VII) to (XIII).

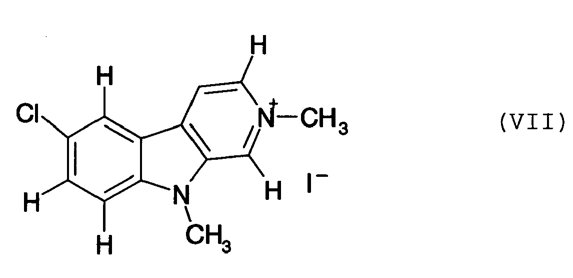

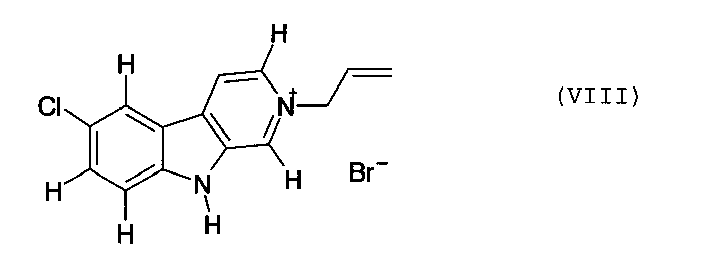

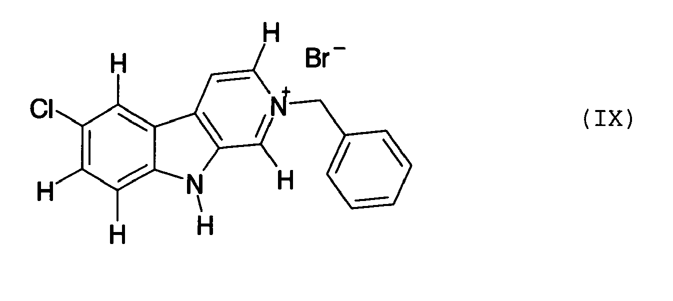

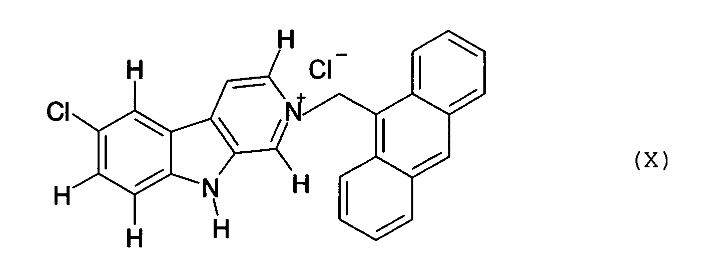

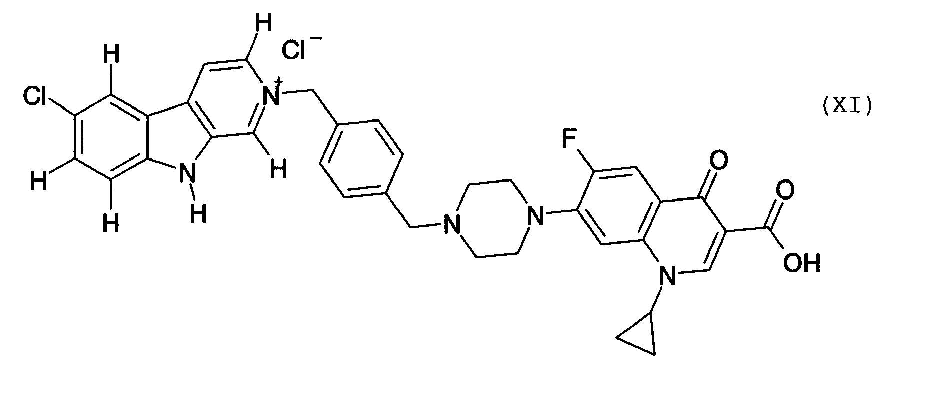

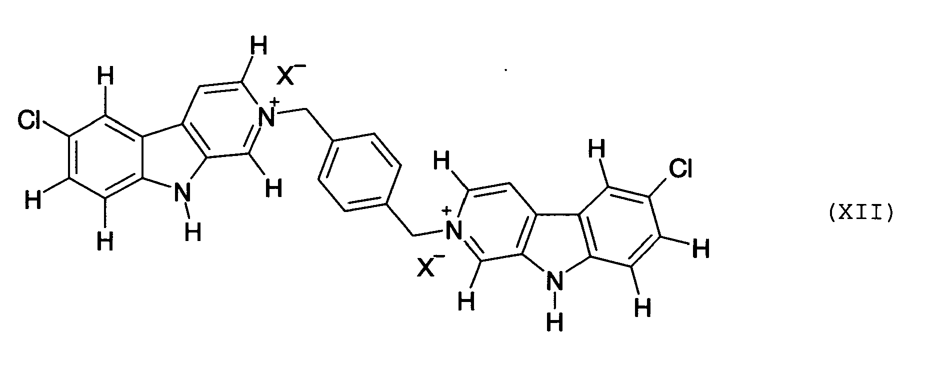

Nostocarboline compounds according to the present invention can be employed in various compositions, e.g. biocides, herbicides, protective coats, paints and the like. Said compositions comprise a suitable carrier, e.g a polymer matrix, a polymer emulsion and the nostocarboline compound. Optionally said compositions may comprise more than one carrier and at least one more bioactive factor different from the nostocarboline compound. The compositions may also comprise two or more different carboline compounds according to the present invention.

As mentioned above the compound according to the present invention are useful to prevent the formation of a biofilm on water exposed surfaces. In order to achieve this, the compound according to the present invention may be employed by coating the submerged structure, for example with a paint or a protective coat containing the required effective amount in such a way that the said compound is released at a sufficiently high level so that the protected surface area is not colonized by microorganisms causing the typical biofouling damages during the desired service life of the antifouling coating. Such aquatic structures are for example ships, oil platforms, seawater pipelines, swimming pools, cooling water devices, turbines, air conditioning systems, cooling devices, submarines, marine infrastructure such as piers, etc.

Antifouling agents are usually used in the form of a protective coat or paint which is applied on the surface which is to be protected against fouling. In closed systems the antifouling agent according to the present invention may either be applied on the submerged surfaces or it may be directly added to the water. Since to some extent the antifouling agent is water-soluble, the direct adding of small amounts of antifouling agent as powder to e.g. a swimming pool is a particularly simple way to achieve antifouling protection.

Especially good results are achieved if the required effective amount of the compound according to the present invention is in the surface layer of the aquatic structure in such a way that the structure is protected by a constant release of the said compounds, such as by a polymer film comprising the compound according to the present invention.

If the surface to be protected is a polymer, e.g. polyvinylchloride (PVC), the compound according to the present invention may be given into the extruder mass, which ensures an optimal distribution of said compound. Further, if due to aging of the polymer fissures are present in the polymer, said fissures are protected as well. Nostocarboline compounds according to the present invention can also be mixed with a polymer emulsion.

The compound according to the present invention may be bound covalently to a polymer matrix, such as methacrylic acid/methylmethacrylate co-polymer matrix. The compound may be bound to said polymer, e.g. PVC, via a linker as defined above. Said linker may additionally comprise hydrolytically unstable bonds, e.g. ester groups.

The amount of compound according to the present invention varies according to the specific compound used, the identity of the fouling organism to be controlled, the water temperature, the mode of contact and the like. Preferably the compound is used in a concentration range of 0.1 to 100 µM, most preferably 1 to 75 µM. The best result has been obtained in a concentration range of 30 to 50 µM.

Due to their excellent efficiency, their selectivety and the cost-effective method for producing the nostocarboline or by K₂Cr₂O₇: Aiello, A.; Fattorusso, E.; Magno S.; Mayol, L. Tetrahedron 1987, 43, 5929-5932.). was converted to 6-Cl-norharmane (Nakano, K.; Suyama, K.; Fukazawa, H.; Uchida, M.; Wakabayashi, K.; Shiozawa, T.; Terao, Y. Mutation Research 2000, 470, 141-146. Ponce, M. A.; Tarzi, O. I.; Erra-Balsells, R. J. Heterocycl. Chem. 2003, 40, 419-426) using a modified literature procedure (8). This compound was then treated with MeI in iPrOH at reflux for 4 hours and the corresponding, bright yellow, quaternized compound 1 was isolated in excellent yield. The resonances in the ¹H-NMR spectrum of nostocarboline are slightly dependant on concentration and, for the synthetic sample 1, the counterion (I⁻, Cl⁻, CF₃CO₂⁻) (For example, H-7 and H-8 became isochronous for 1 iodide, 600 MHz, 23°C, c = 21 mM in CD₃OD). Overall, spectroscopic data (UV, ¹H-NMR, ¹³C-NMR) of the synthetic sample **1** were in very good agreement to those of nostocarboline. The final proof of structure was delivered by mixing natural and synthetic samples, which were thus shown to be identical by ¹H-NMR spectroscopy (8). The structure of nostocarboline was thus definitely established as 1 by chemical synthesis.

**Table 1.** NMR spectroscopic data of nostocarboline (CD₃OD, 600 MHz). Lines denote key *J* couplings or NOEs.
compounds according to the present invention they can be widely employed as biocidal agents. The high selectivity prevents unwanted toxicity against beneficial organisms. Therefore, nostocarboline compounds according to the present invention are very useful antifouling agents or herbicides. Nostocarboline compounds have an very good stability at room temperature and thus a very good shelf-life. In addition, the storage does not require any costly measures such as cooling.

Surfaces which are submerged are most susceptible to biofouling and the damages associated thereto. The nostocarboline compounds according to the present invention are particulary useful to prevent biofouling on submerged surfaces.

### Example 1

### Synthesis of nostocarboline

### Scheme 1. Synthesis of nostocarboline (1).

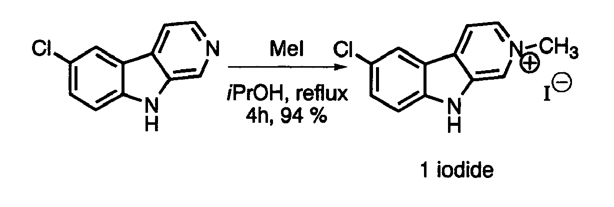

Norharmane (Norharmane was prepared from tryptophan via the tetrahydro-β-carboline (Lippke, K. P.; Schunack, W. G.; Wenning, W.; Müller, W. E.; J. Med. Chem. 1983, 26, 499-503) followed by oxidation by SeO₂ (Hagen, T. J.; Skolnick, P.; Cook, J. M. J. Med. Chem. 1987, 30, 750-753)

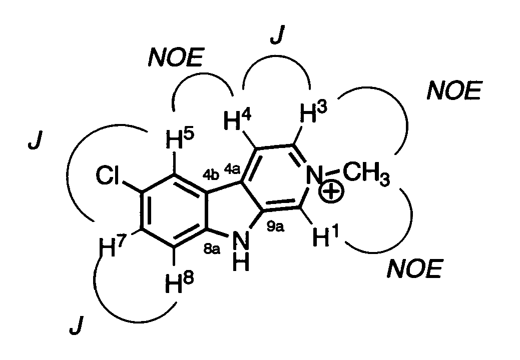

| Position | δ_{H} | δ_{C} | J (Hz) | HMBC¹ | NOE |
|---|---|---|---|---|---|
| 1 | 9.23 | 132.4 | br. s. | C-3, C-4a | *N*-Me |
| 3 | 8.52 | 134.6 | *dd,* 6.4 (H-4), 0.9 (H-1) | C-4a, C-1, C-4 | H-4 |
| 4 | 8.68 | 119.3 | *d*, 6.4 (H-3) | C-9a | H-5, H-3 |
| 4a | | 134.0 | | | |
| 4b | | 122.1 | | | |
| 5 | 8.49 | 123.8 | *dd,* 2.0 (H-7), 0.8 (H-8) | C-6, C-7, C-8a | H-4 |
| 6 | | 128.4 | | | |
| 7 | 7.80 | 133.8 | *dd,* 8.9 (H-8), 2.0 (H-5) | C-8a | |
| 8 | 7.77 | 115.5 | *dd,* 8.9 (H-7), 0.8 (H-5) | C-4b, C-6 | |
| 8a | | 145.5 | | | |
| 9a | | 138.4 | | | |
| *N*-Me | 4.53 | 48.8 | s | C-1, C-3 | H-1, H-3 |

| | | | | | |
|---|---|---|---|---|---|
| ¹ Denotes HMBC correlations from proton at position X to the corresponding carbon. | | | | | |

Chemicals were generally purchased from ABCR, Acros, Aldrich or Fluka. Norharmane was prepared according to the literature (Norharmane was prepared from tryptophan via the tetrahydro-β-carboline (Lippke, K. P.; Schunack, W. G.; Wenning, W.; Müller, W. E.; J. Med. Chem. 1983, 26, 499-503) followed by oxidation by SeO₂ (Hagen, T. J.; Skolnick, P.; Cook, J. M. J. Med. Chem. 1987, 30, 750-753) or by K₂Cr₂O₇: Aiello, A.; Fattorusso, E.; Magno S.; Mayol, L. Tetrahedron 1987, 43, 5929-5932). 6-Chloroharmane was prepared using a modified literature procedure (see supporting information) (Nakano, K.; Suyama, K.; Fukazawa, H.; Uchida, M.; Wakabayashi, K.; Shiozawa, T.; Terao, Y. Mutation Research 2000, 470, 141-146. Ponce, M. A.; Tarzi, O. I.; Erra-Balsells, R. J. Heterocycl. Chem. 2003, 40, 419-426) 2-Methylnorharmane was prepared according to van Tamelen et al. (Van Tamelen et al. proposed to have isolated the 6-Cl species, but did not provide sufficient characterization: Van Tamelen, E. E.; Haarstad, V. B.; Orvis, R. L. Tetrahedron 1968, 24, 687-704.). HPLC-ESI-MS spectra were obtained on a LC-MS (LCQ Duo mass spectrometer, Finnigan Thermoquest). ¹H and ¹³C NMR spectra were recorded using either Varian Gemini (300 MHz (¹H) or 75 MHz (¹³C)), Varian Mercury (300 MHz (¹H) or 75 MHz (¹³C)), Bruker AV600 600 MHz (¹H) or 150 MHz (¹³C)) FT spectrometers at ambient temperature, chemical shifts □ are given in ppm, coupling constants J are in Hz. IR spectra were recorded as a KBr pellet using a Perkin Elmer RX I FT-IR spectrometer, absorptions are given in cm⁻¹. Mass spectra were recorded by the MS Service of the Laboratorium für Organische Chemie der ETH Zürich on an IonSpec Ultima 4.7 FT-ICR spectrometer using MALDI, fragment ions are given in m/z with relative intensities (%) in parentheses.

**Culture of *Nostoc* 78-12A.** The axenic strain *Nostoc* 78-12A (identical to *Anabaena* 78-12A and ATCC 43238) was obtained from C. P. Wolk (MSU, East Lansing, USA) as a liquid culture. It was grown in glass tower-type reactors as previously described (Bister, B.; Keller, S.; Baumann, H. I.; Nicholson, G.; Weist, S.; Jung, G.; Süssmuth, R. D.; Jüttner, F. J. Nat. Prod. 2004, 67, 1755-1757). The yield was 2.0 g wet biomass per liter. During centrifugation in a flow through centrifuge, the cells lyse to a great extent and lose nostocarboline to the medium. Therefore, short centrifugation steps are required to retain sufficient amounts of nostocarboline in the cells. The ¹⁵N-labeled compound was obtained from cultures in 300-ml Erlenmeyer flasks by replacing the naturally labeled nitrate of the mineral medium by ¹⁵N-enriched nitrate (98 atom %, Cambridge Isotope Laboratories, Inc., Andover MA, USA).

**Isolation of Nostocarboline.** The frozen wet biomass (8.8 g) of *Nostoc* was extracted with methanol. Methanol was added in quantities to obtain a final concentration of 60% aq. methanol (MeOH:H₂0, 60:40: v:v) assuming 85% water in the wet biomass. The extract was brought to dryness on a rotary evaporator at 40°C under reduced pressure. To remove the lipid by-products, the residue was washed with CH₃CN. The bioactive factor was then extracted 4 times with 1-ml portions of 95% aq. CH₃CN (CH₃CN:H₂O, 95:5, v:v). The yellow extract was separated by HPLC on a Polyamine II column (250 x 4.6 mm i.d., S-5 µm, 12 nm, YMC Europe GmbH) using the solvent 30% aq. CH₃CN at pH 4 (trifluoroacetic acid) and reading the absorption at 306 nm. Nostocarboline eluted at 2.5 min. The natural anion (counterion) is not known. The concentration of nostocarboline was determined in a standing culture of *Nostoc* (12 µg chlorophyll a / ml) to be 0.4 µg nostocarboline/ µg chlorophyll a.

### Example 2

**Nostocarboline iodide (1)**. 6-Cl-norharmane (70 mg, 0.35 mmol) was dissolved in iPrOH (2 ml). MeI (45 µl, 0.70 mmol, 2 equiv.) was added at room temperature and the resulting suspension heated to reflux for 4 hours. The resulting yellow suspension was then cooled to room temperature and filtered. The filtrate was washed with iPrOH and dried under high vacuum. Recrystallization from MeOH/*i*PrOH gave the title compound 1 iodide (113 mg, 0.33 mmol, 94 %).

Bright yellow, thin needles (MeOH): mp > 215°C. UV (MeOH) λₘₐₓ (log ε) 254 (3.68), 307 (3.46), 385 (2.81). IR (KBr) 2979, 1644, 1572, 1518, 1492, 1451, 1323, 1284, 1152, 1067, 879, 834, 806. ¹H- and ¹³C-NMR see Table 1. MS (MALDI, matrix: 3-hydroxypicolinic acid) 235.1 (7), 233.1 (15), 219.0 (39), 218.0 (18), 217.0 (100). HIRES-MALDI *m*/*z* 217.0523 (calcd. for C₁₂H₁₀³⁵ClN₂ 217.0527, □ = 1.84 ppm). *Anal.* C 41.56%, H 2.99%, N 8.00%, calcd for C₁₂H₁₀N₂ClI, C 41.83%, H 2.93%, N 8.13%.

### Example 3

### 2-Allyl-nornostocarboline bromide.

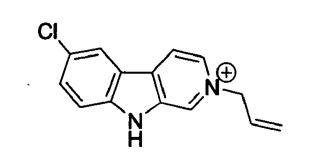

6-Cl-norharmane (50 mg 0.25 mmol) was dissolved in iPrOH (4ml). Allylbromide (32 µl, 0.37 mmol, 1.5 equiv.) was added at room temperature and the reaction mixture heated to reflux for 20 h. The resulting light brown solution was evaporated. The light brown solid was dissolved in boiling chloroform, cooled to room temperature and Et₂O was added. Thin needles crystallized that were collected by filtration. The filtrate was washed with EtOAc and dried under high vacuum to give the title compound (36 mg, 0.11 mmol, 45 %). Light brown, thin needles. M.p. (Chloroform / Et₂O) 174-177°C. UV (MeOH) λₘₐₓ (log ε) 245 (4.21), 298 (3.98), 377 (3.36). IR (ATR) 3007m, 2949m, 2855m, 1905w, 1813w, 1745s, 1651s, 1489s, 1285s, 951s, 806s. ¹H-NMR 9.31 (s, 1H), 8.71 (d, *J =* 6.6, 1H), 8.58 (*dd, J₁ =* 6.6 *J₂* = 1.1, 1H), 8.47 (d, *J =* 1.1, 1H), 7.78 (s, 2H), 6.33-6.20 (m, 1H), 5.55 (d, *J =* 1.4, 1H), 5.51 (d, *J*₁= 5.1, 1H), 5.39 (d, J = 6.3, 2 H). ¹³C-NMR 143.5, 136.7, 133.3, 133.2, 133.1, 132.3, 130.4, 128.0, 123.2, 122.4, 121.2, 119.0, 114.9, 64.1. MS 245 (32), 243 (100), 209 (6), 204 (10), 202 (12).

### Example 4

### 2-Benzyl-nornostocarboline bromide.

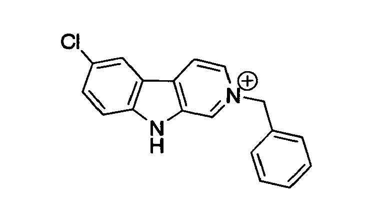

6-Cl-norharmane (50 mg, 0.25 mmol) was dissolved in iPrOH (4ml). BnBr (43µl, 0.37 mmol, 1.5 equiv.) was added at room temperature and the reaction mixture heated to reflux for 24 h. The yellow solution was evaporated and the resulting yellow solid was dissolved in chloroform under reflux. The solution was then cooled to room temperature and Et₂O added. The product crystallized as thin yellow needles and was collected by filtration. The crystlas were washed with EtOAc and dried under high vacuum to give the title compound (54 mg, 0.14 mmol, 60 %). Bright yellow, thin needles (Chloroform / Et₂O). M.p. 121 - 125 °C. UV (MeOH) λₘₐₓ (log ε) 211 (4.22), 260 (4.3), 310 (4.2), 389 (3.4). IR (ATR) 3399w *br.,* 3057m, 2970m, 1643m 1491s, 1284s, 730s. ¹H-NMR 9.41 (s, 1H), 8.71-8.67 (m, 2H), 8.45 (s, 1H), 7.89 (s, 1H), 7.76 (d, *J* = 1.25, 2H), 7.55-7.44 (m, 5H), 5.96 (s, 2H). ¹³C-NMR 143.2, 136.4, 135.2, 133.3, 132.9, 130.3, 130.2, 130.2, 129.7, 127.8, 123.0, 120.8, 119.1, 114.7, 79.2, 65.1. MS 295.2 (30), 293.2 (100), 259.2 (7).

### Example 5

### 8-Chloro-nostocarboline iodide.

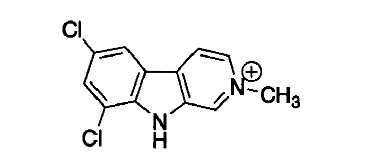

6, 8-Dichloro-norharmane (50 mg, 0.21 mmol) was dissolved in iPrOH (4 mL). MeI (26µl, 0.42mmol 2 equiv.) was added at room temperature and heated the suspension was heated to reflux for 4 h. The resulting yellow solution was evaporated and the residue recrystallised from iPrOH / MeOH to give the title compound (40 mg, 0.11 mmol, 52 %). Bright yellow solid. m.p > 220 °C. UV (MeOH) λₘₐₓ (log ε) 219 (4.3), 255 (4.3), 285 (4.0), 304 (3.9), 389 (3.4). IR (ATR) 3400*m*, 3022*m*, 2051*w*, 1983*w*, 1791*w*, 1652*m*, 1493*s*, 1277*s*, 845*s*, 737*s*, 671*w*. ¹H-NMR 9.24 (*s*, 1H), 8.72 (*d*, *J* = 6.5, 1 H), 8.59 (d, *J =* 6.5, 1H), 8.47 (d, J = 1.6, 1 H), 7.91 (*d*, *J* = 1.6, 1H), 4.57 (*s*, 3H). MS 255.1 (8), 253.1 (64), 251.1 (100), 243.2 (40).

### Example 6

The following example shows the biocidal activity of different nostocarboline compounds against three selected microorganisms:
- 2 cyanobacteria *(Microcystis aeruginosa* PCC 7806 (Ma);*Synechococcus* PCC 6911 (Sc))
- 1 green alga (*Kirchneriella contorta* SAG 11.81 (Kc))

The microorganisms were grown in commercial 96-well microtiter plates; 200 µl cell suspension was added to each well having an OD_{675 nm} of approximately 0.1 at the beginning. This value of about 0.1 was subtracted from all subsequent measurements. The microorganisms were grown on a cyanobacterial medium as described in Jüttner et al. (Jüttner F., Leonhardt J., Möhren S., 1983, Journal of General Microbiology 129, p. 407-412) at 20°C under continuous light from fluorescent tubes (7 µmol m⁻² s⁻¹).

Measurements of OD_{675 nm} were performed daily with an Absorbance Microplate Reader (SpectraMax; Molecular Devices Corporation).

The different compounds were added on the 5^{th} day (marked by an arrow) in 20 µl, compounds dissolved in 2% DMSO (end concentration 0.2% DMSO).

Table 2 shows which compounds were tested with which microorganism. Briefly, 9 compounds and 1 control were tested for Ma and Sc, 3 compounds and 1 control were tested for Kc.

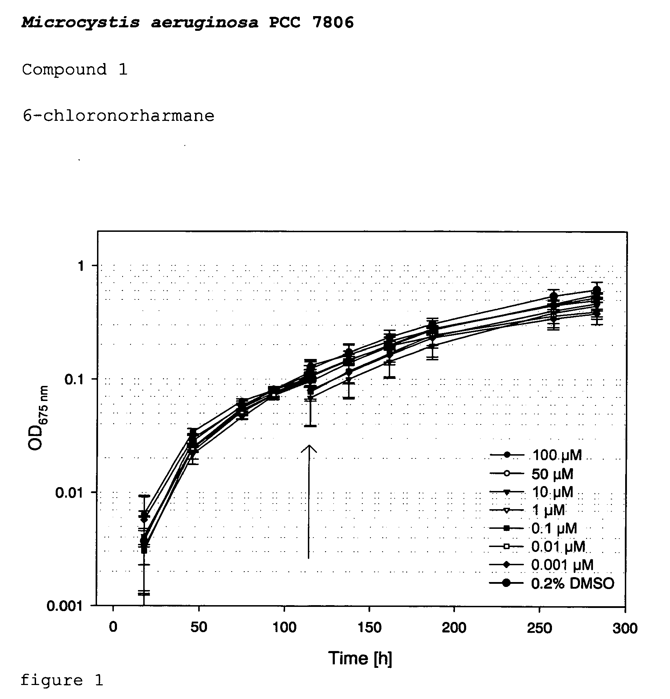

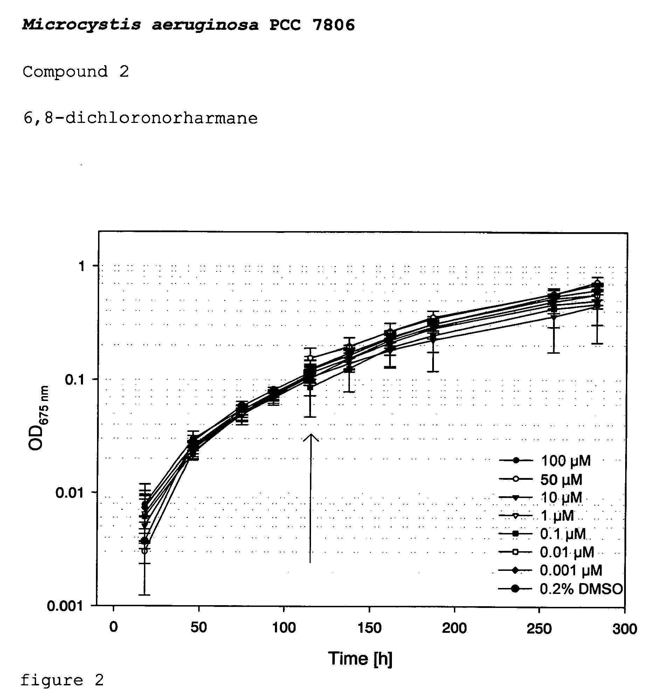

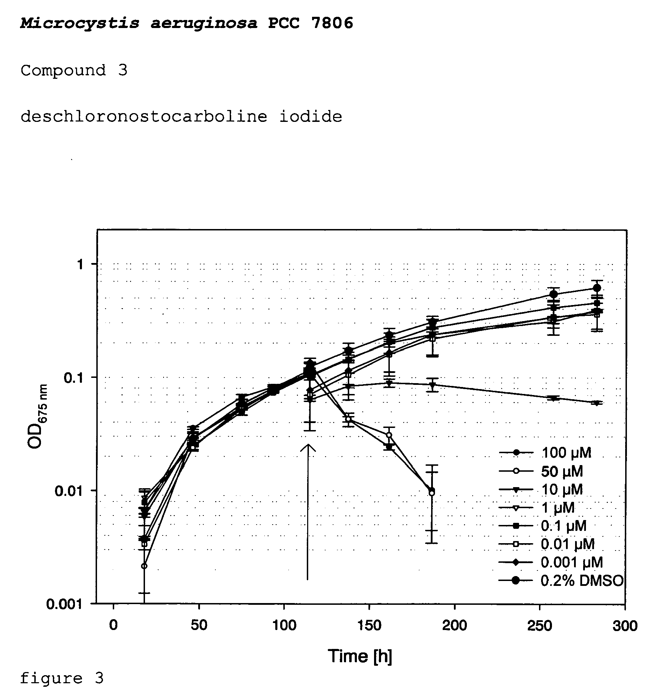

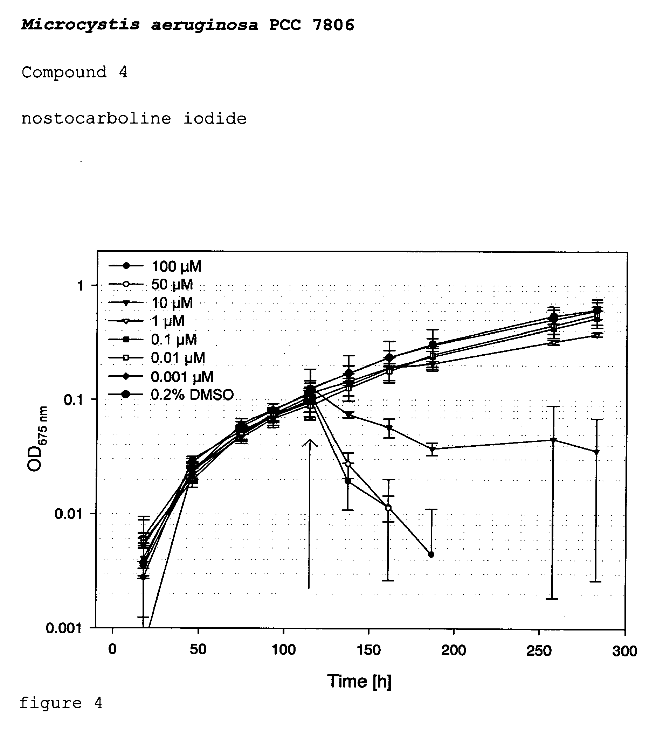

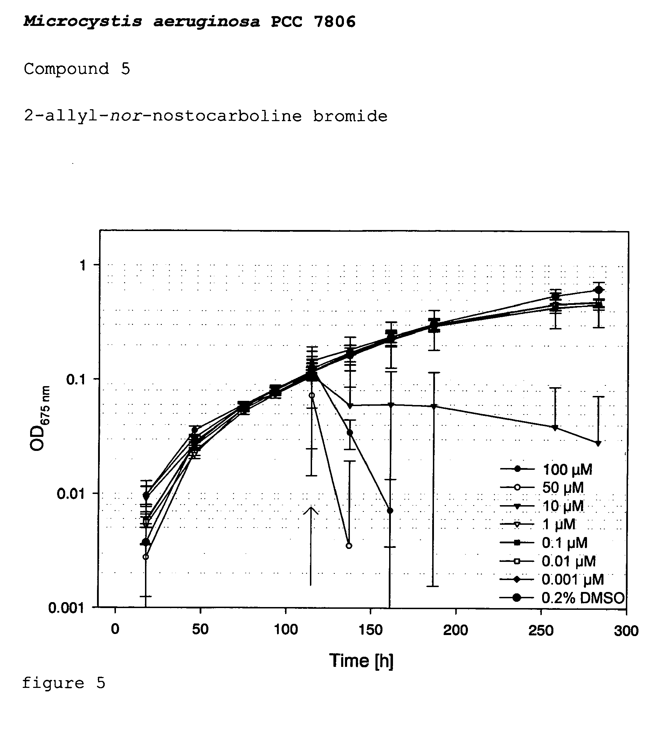

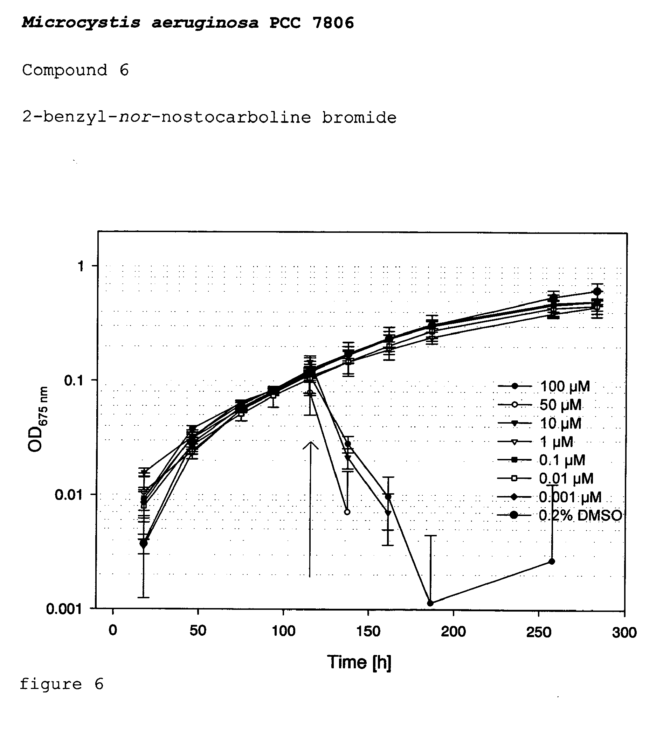

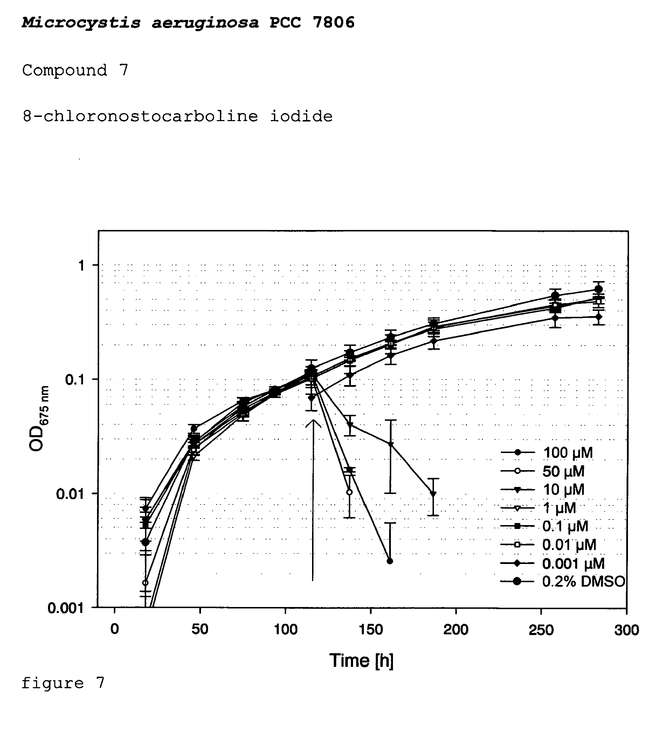

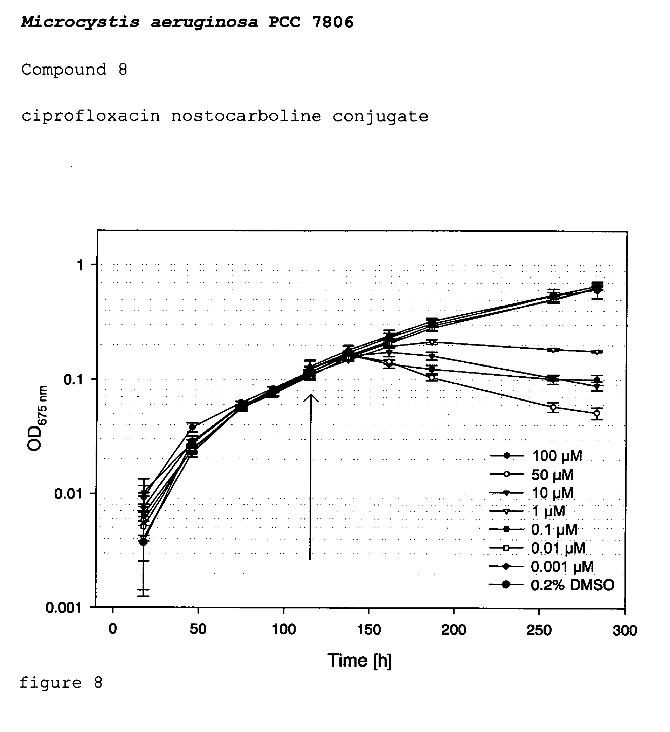

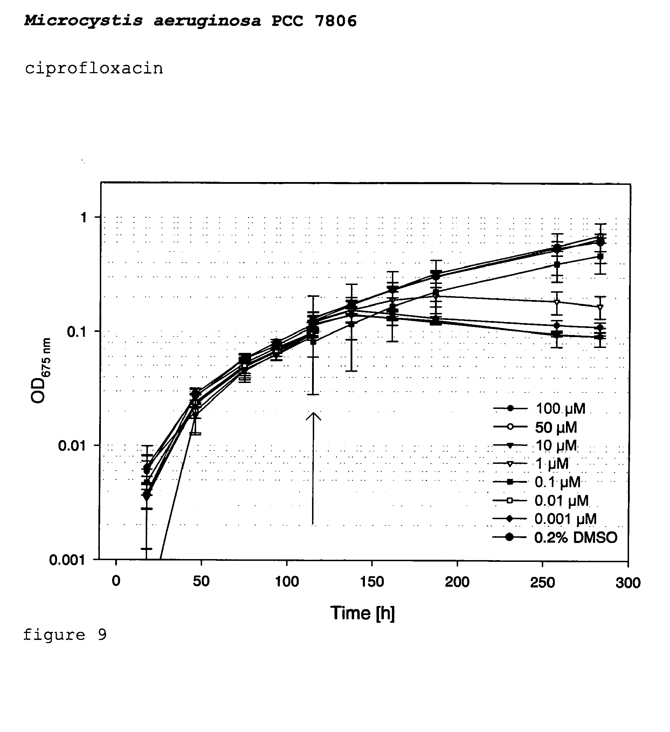

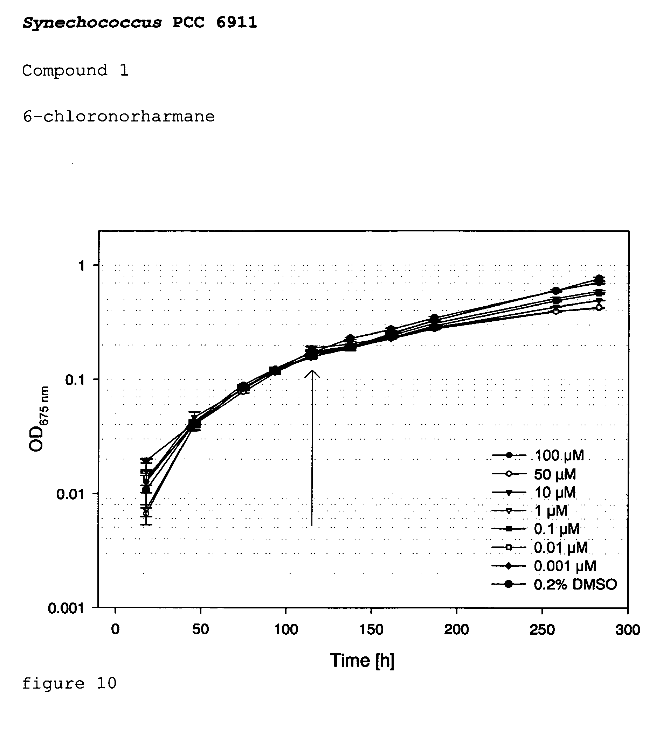

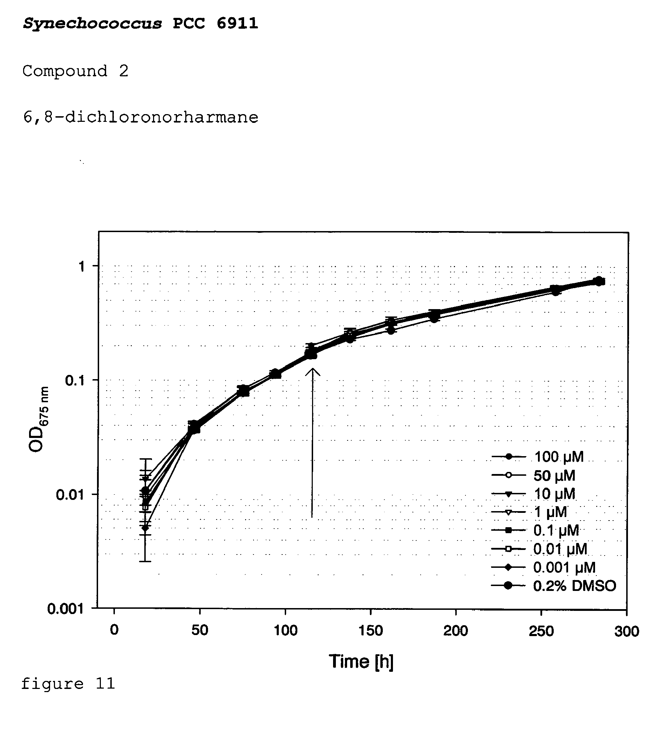

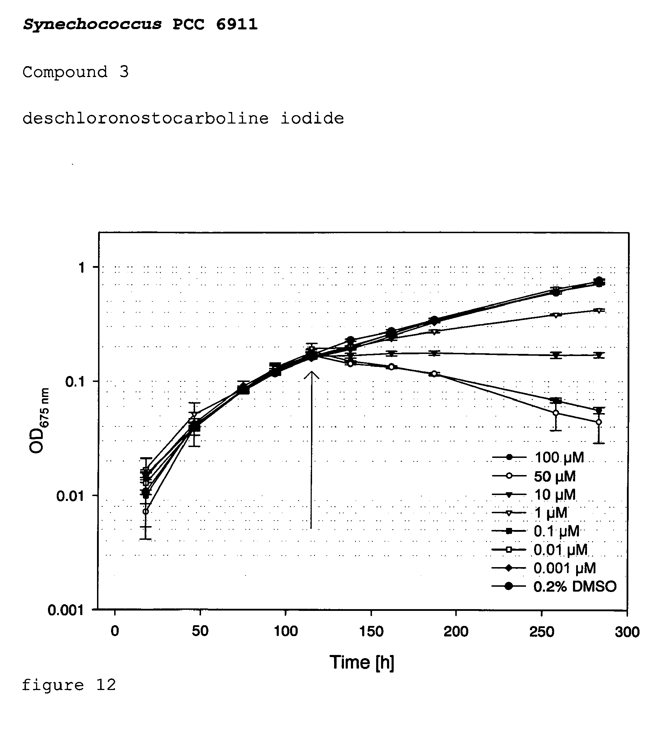

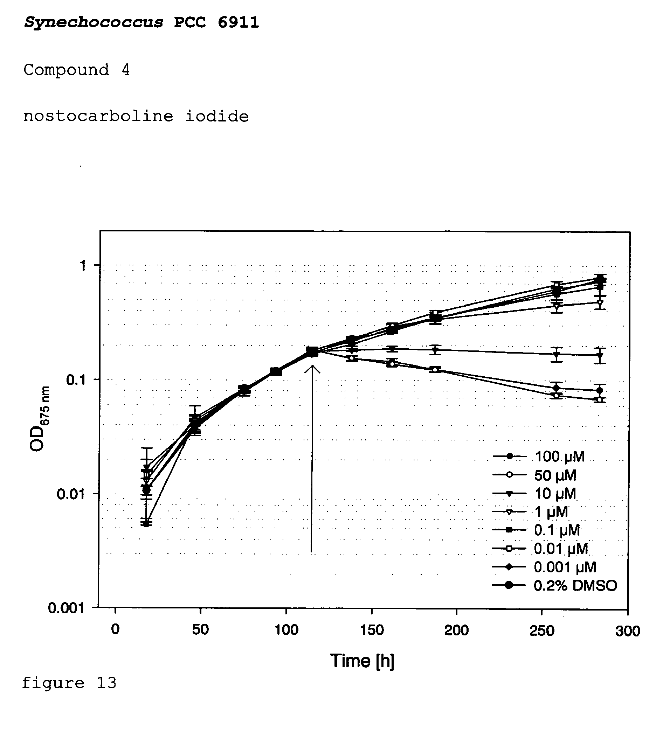

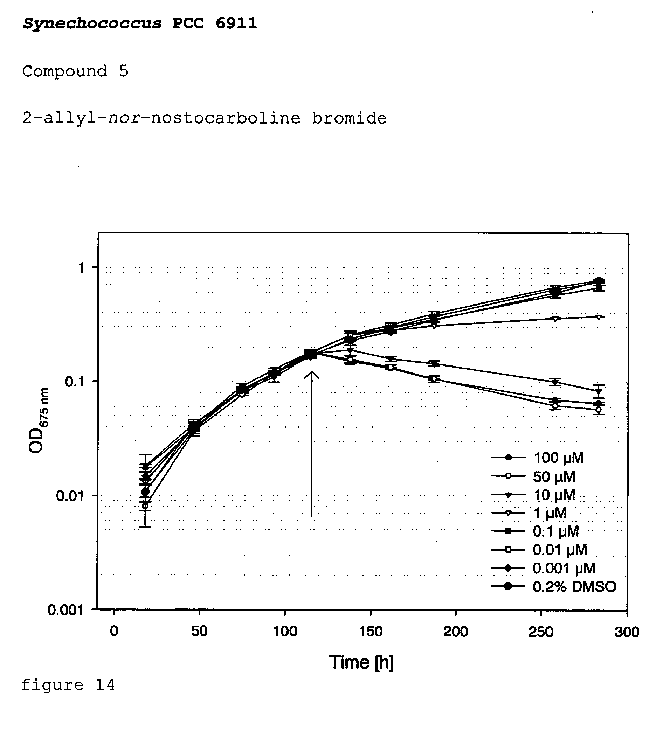

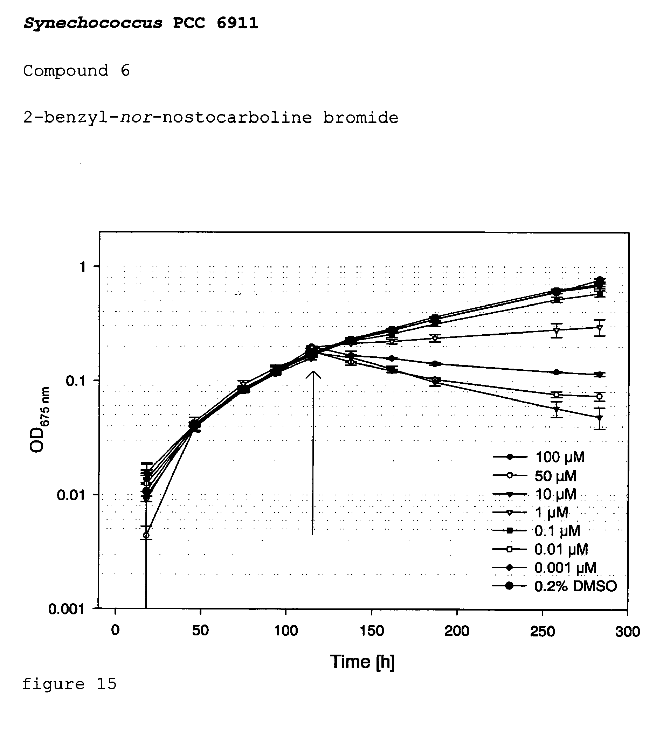

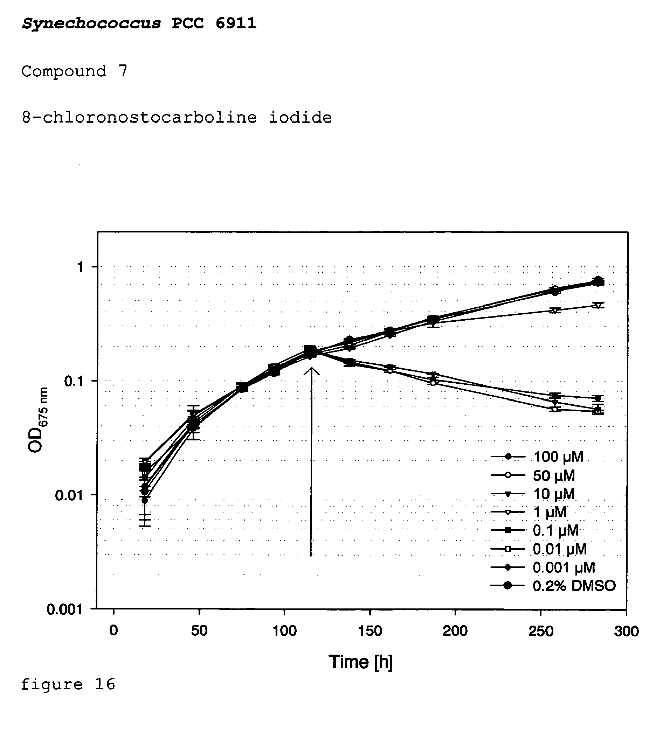

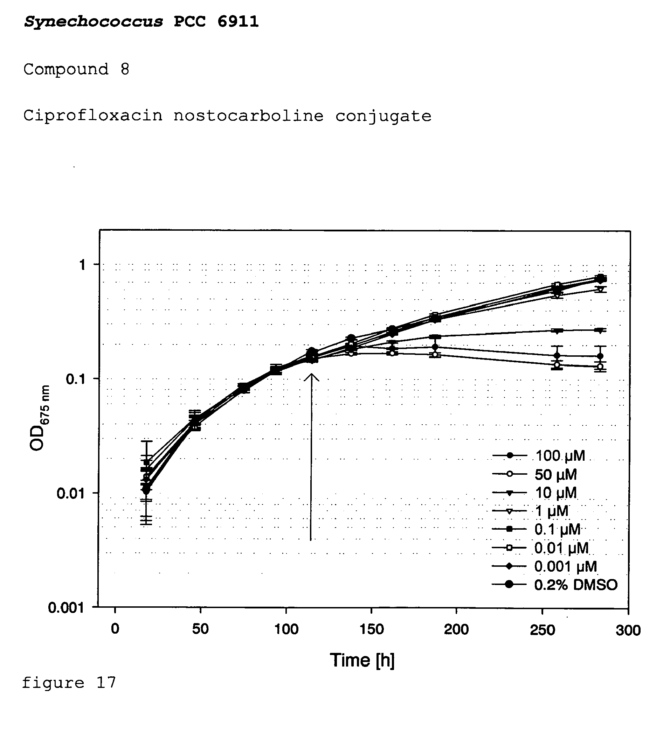

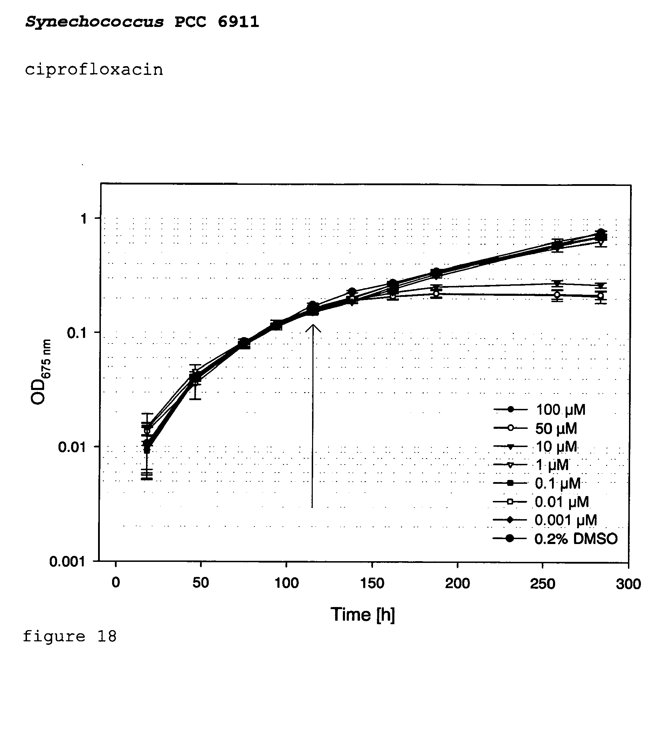

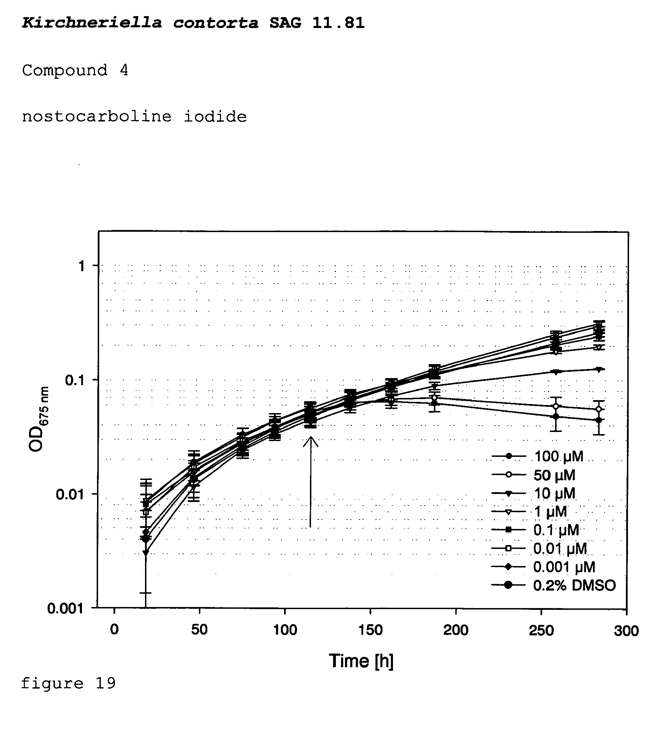

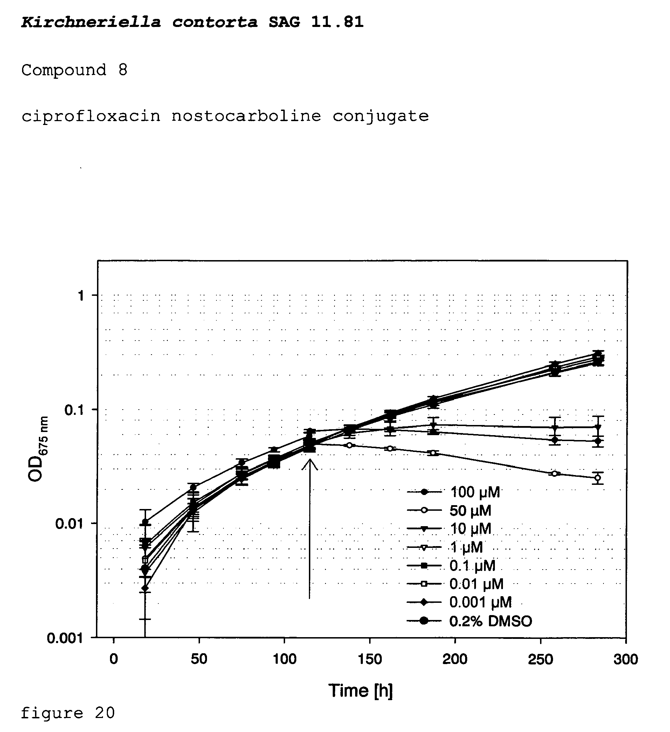

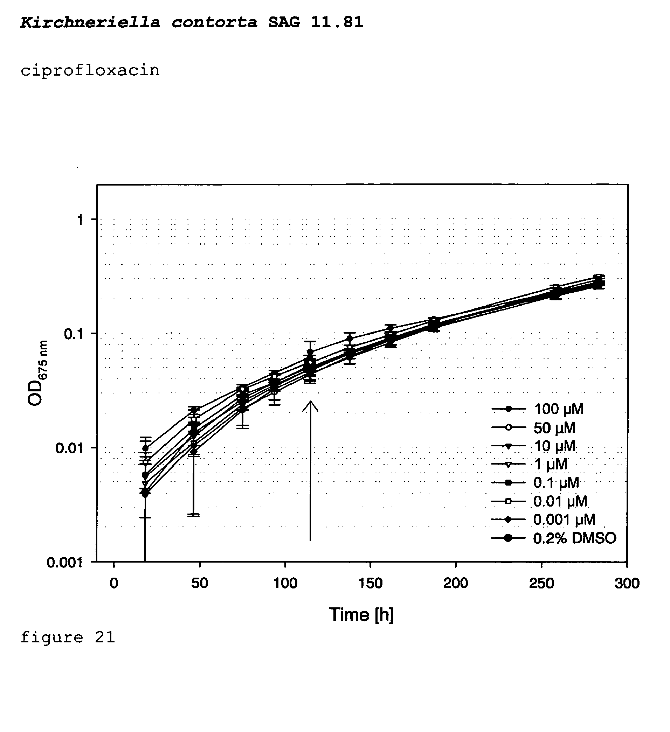

The growth curves are shown in figures 1 to 21.

**Table 2**

| Compound | Ma | Sc | Kc |
|---|---|---|---|
| 6-chloronorharmane | + | + | |
| 6,8-dichloronorharmane | + | + | |
| deschloronostocarboline iodide | + | + | |
| nostocarboline iodide | + | + | + |
| 2-allyl-*nor*-nostocarboline bromide | + | + | |
| 2-benzyl-*nor*-nostocarboline bromide | + | + | |
| 8-chloronostocarboline iodide | + | + | |
| ciprofloxacin nostocarboline conjugate | + | + | + |
| ciprofloxacin | + | + | + |
| 2% DMSO (control) | + | + | + |

Each compound was tested in 7 concentrations (100 µM; 50 µM; 10 µM; 1 µM; 0.1 µM; 0.01 µM; 0.001 µM), each concentration was done in triplicate and each control was done in 12 replicates.

## Claims

1. Use of a nostocarboline compound of the formula (I) 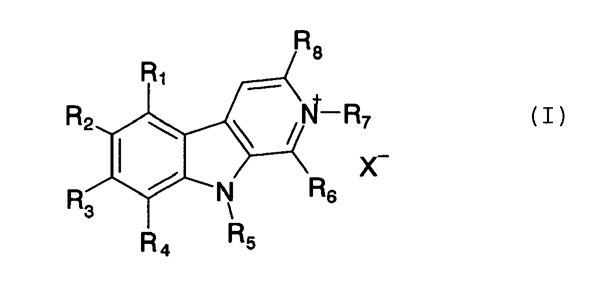 wherein
R₁, R₃, R₄ and R₆ are independently selected from the group consisting of H, F, Cl, Br, I, a saturated or unsaturated, substituted or unsubstituted C₁-C₂₀ aliphatic residue with straight or branched chain optionally having one or more heteroatom at the beginning or in the chain, and a saturated or unsaturated, substituted or unsubstituted C₁-C₂₀ aliphatic residue with straight or branched chain comprising a carbocyclic or heterocyclic residue;
R₂ is selected from the group consisting of H and Cl;
R₅ is selected from the group consisting of H, a saturated or unsaturated, substituted or unsubstituted C₁-C₂₀ aliphatic residue with straight or branched chain optionally having one or more heteroatom in the chain, and a saturated or unsaturated, substituted or unsubstituted C₁-C₂₀ aliphatic residue with straight or branched chain comprising a carbocyclic or heterocyclic residue;
R₇ is selected from the group consisting of a saturated or unsaturated, substituted or unsubstituted C₁-C₂₀ aliphatic residue with straight or branched chain optionally having one or more heteroatom in the chain, a saturated or unsaturated, substituted or unsubstituted C₁-C₂₀ aliphatic residue with straight or branched chain comprising a carbocyclic or heterocyclic residue and optionally having one or more heteroatoms in the chain, and a linker bound to a bioactive factor;
R₈ is selected from the group consisting of H, a saturated or unsaturated, substituted or unsubstituted C₁-C₆ aliphatic carboxylic acid with straight or branched chain, a saturated or unsaturated, substituted or unsubstituted C₁-C₆ aliphatic carboxylic ester with straight or branched chain, a saturated or unsaturated, substituted or unsubstituted C₁-C₆ aliphatic carboxylic amide with straight or branched chain, and a C₃-C₂₀ carbocyclic residue; and
X⁻ is a negatively charged ion,
as a biocidal agent.

2. Use of the Nostocarboline compound according to claim 1 wherein
R₁, R₃, R₄ and R₆ are independently selected from the group consisting of H, F, Cl, Br, I, and unsubstituted, saturated or unsaturated C₁-C₅ aliphatic residues;
R₂ is selected from the group consisting of H and Cl;
R₅ is selected from the group consisting of H, and unsubstituted, saturated or unsaturated C₁-C₅ aliphatic residues;
R₇ is selected from the group consisting of unsubstituted, saturated or unsaturated C₁-C₅ aliphatic residues, phenyl, benzyl, naphtyl, anthranyl residues, and a linker bound to a nostocarboline compound as claimed in claim 1, Ciprofloxacin, Fosfomycin, Vancomycin, and Linezolid;
R₈ is selected from the group consisting of H, a saturated or unsaturated, substituted or unsubstituted C₁-C₆ aliphatic carboxylic acid with straight or branched chain, a saturated or unsaturated, substituted or unsubstituted C₁-C₆ aliphatic carboxylic ester with straight or branched chain, a saturated or unsaturated, substituted or unsubstituted C₁-C₆ aliphatic carboxylic amide with straight or branched chain, and a C₃-C₂₀ carbocyclic residue; and
X⁻ is an ion selected from the group consisting of F, Cl, Br, I, CF₃CO₂, PO₄, HPO₄, H₂PO₄, SO₄, HSO₄, NO₃, OH, citrate, fumarate, tartrate aspartate, and glutamate,
as biocidal agent.

3. Use of the Nostocarboline compound according to claim 1 or 2 wherein
R₂ is Cl
as biocidal agent.

4. Use of the Nostocarboline compound according to any of claim 1 to 3 wherein
the linker is selected from the group consisting of a saturated or unsaturated, substituted or unsubstituted C₁-C₂₀ alkylen residue with straight or branched chain optionally having one or more heteroatom in the chain, and a saturated or unsaturated, substituted or unsubstituted carbocyclic or heterocyclic residue bound to a bioactive factor
as biocidal agent.

5. Use of the Nostocarboline compound according to any of claim 1 to 4 wherein
the bioactive factor is selected from the group consisting of a nostocarboline compound as claimed in claim 1, Ciprofloxacin, Fosfomycin, Vancomycin, and Linezolid
as biocidal agent.

6. Use of the Nostocarboline compound according to any of claim 1 to 5 wherein
the bioactive factor is a nostocarboline compound as claimed in claim 1 and forms a dimer
as biocidal agent.

7. Use of a biocidal agent according to claim 1 to 6 as an antifouling agent.

8. Use of a biocidal agent according to any of claim 1 to 6 as a herbicide.

9. Use of a biocidal agent according to any of claim 1 to 6 in protective coats for surfaces submerged in water.

10. Nostocarboline compound of the formula (I) 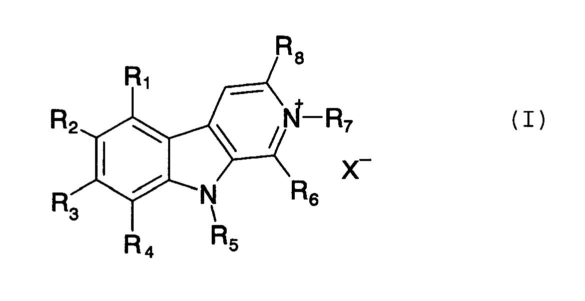 wherein
R₁, R₃, R₄ and R₆ are independently selected from the group consisting of H, F, Cl, Br, I, a saturated or unsaturated, substituted or unsubstituted C₁-C₂₀ aliphatic residue with straight or branched chain optionally having one or more heteroatom at the beginning or in the chain, and a saturated or unsaturated, substituted or unsubstituted C₁-C₂₀ aliphatic residue with straight or branched chain comprising a carbocyclic or heterocyclic residue;
R₂ is Cl;
R₅ is selected from the group consisting of H, a saturated or unsaturated, substituted or unsubstituted C₁-C₂₀ aliphatic residue with straight or branched chain optionally having one or more heteroatom in the chain, and a saturated or unsaturated, substituted or unsubstituted C₁-C₂₀ aliphatic residue with straight or branched chain comprising a carbocyclic or heterocyclic residue;
R₇ is selected from the group consisting of a saturated or unsaturated, substituted or unsubstituted C₁-C₂₀ aliphatic residue with straight or branched chain optionally having one or more heteroatom in the chain, a saturated or unsaturated, substituted or unsubstituted C₁-C₂₀ aliphatic residue with straight or branched chain comprising a carbocyclic or heterocyclic residue and optionally having one or more heteroatoms in the chain, and a linker bound to a bioactive factor;
R₈ is selected from the group consisting of H, a saturated or unsaturated, substituted or unsubstituted C₁-C₆ aliphatic carboxylic acid with straight or branched chain, a saturated or unsaturated, substituted or unsubstituted C₁-C₆ aliphatic carboxylic ester with straight or branched chain, a saturated or unsaturated, substituted or unsubstituted C₁-C₆ aliphatic carboxylic amide with straight or branched chain, and a C₃-C₂₀ carbocyclic residue; and
X⁻ is a negatively charged ion.

11. Nostocarboline compound according to claim 10 wherein
R₁, R₃, R₄ and R₆ are independently selected from the group consisting of H, F, Cl, Br, I, and unsubstituted, saturated or unsaturated C₁-C₅ aliphatic residues;
R₂ is Cl;
R₅ is selected from the group consisting of H, and unsubstituted, saturated or unsaturated C₁-C₅ aliphatic residues;
R₇ is selected from the group consisting of unsubstituted, saturated or unsaturated C₁-C₅ aliphatic residues, phenyl, benzyl, naphtyl, anthranyl residues, and a linker bound to a nostocarboline compound as claimed in claim 1, Ciprofloxacin, Fosfomycin, Vancomycin, and Linezolid;
R₈ is selected from the group consisting of H, a saturated or unsaturated, substituted or unsubstituted C₁-C₆ aliphatic carboxylic acid with straight or branched chain, a saturated or unsaturated, substituted or unsubstituted C₁-C₆ aliphatic carboxylic ester with straight or branched chain, a saturated or unsaturated, substituted or unsubstituted C₁-C₆ aliphatic carboxylic amide with straight or branched chain, and a C₃-C₂₀ carbocyclic residue; and
X⁻ is an ion selected from the group consisting of F, Cl, Br, I, CF₃CO₂, PO₄, HPO₄, H₂PO₄, SO₄, HSO₄, NO₃, OH, citrate, fumarate, tartrate aspartate, and glutamate.

12. Nostocarboline compound according to claim 10 or 11 wherein
R₁, R₃, R₄ and R₆ are independently selected from the group consisting of H, F, Cl, Br, I, and unsubstituted, saturated or unsaturated C₁-C₅ aliphatic residues;
R₂ is Cl;
R₅ is selected from the group consisting of H, and unsubstituted, saturated or unsaturated C₁-C₅ aliphatic residues;
R₇ is selected from the group consisting of unsubstituted, saturated or unsaturated C₂-C₅ aliphatic residues, phenyl, benzyl, naphtyl, anthranyl residues, and a linker bound to a nostocarboline compound as claimed in claim 1, Ciprofloxacin, Fosfomycin, Vancomycin, and Linezolid;
R₈ is selected from the group consisting of H, a saturated or unsaturated, substituted or unsubstituted C₁-C₆ aliphatic carboxylic acid with straight or branched chain, a saturated or unsaturated, substituted or unsubstituted C₁-C₆ aliphatic carboxylic ester with straight or branched chain, a saturated or unsaturated, substituted or unsubstituted C₁-C₆ aliphatic carboxylic amide with straight or branched chain, and a C₃-C₂₀ carbocyclic residue; and
X⁻ is an ion selected from the group consisting of F, Cl, Br, I, CF₃CO₂, PO₄, HPO₄, H₂PO₄, SO₄, HSO₄, NO₃, OH, citrate, fumarate, tartrate aspartate, and glutamate.

13. Nostocarboline compound according to any of claim 10 to 12 wherein R₁ and R₃ are H.

14. Nostocarboline compound according to claim 10 to 12 wherein R₄ is either H or Cl.

15. Nostocarboline compound according to claim 10 to 12 wherein R₂ and R₄ are Cl.

16. Nostocarboline compound according to claim 10 to 12 wherein R₁, R₃, and R₄ and are H, R₂ is Cl and R₅ and R₇ are methyl residues and X⁻ is I.

17. Nostocarboline compound according to claim 10 to 12 wherein R₁, R₃, R₄ and R₅ are H, R₂ is Cl and R₇ is an allyl residue and X⁻ is Br.

18. Nostocarboline compound according to claim 10 to 12 wherein R₁, R₃, R₄ and R₅ are H, R₂ is Cl and R₇ is a benzyl residue and X⁻ is Br.

19. Nostocarboline compound according to claim 10 to 12 wherein R₁, R₃, R₄ and R₅ are H, R₂ is Cl and R₇ is an anthranyl residue and X⁻ is Cl.

20. Nostocarboline compound according to claim 10 to 12 wherein R₁, R₃, R₄ and R₅ are H, R₂ is Cl and R₇ is ciprofloxazin comprising dimethylbenzene as linker and X⁻ is Cl.

21. Nostocarboline compound according to claim 10 to 12 wherein R₁, R₃, R₄, R₅, R₆ and R₈ are H, R₂ is Cl, R₇ is methyl and X⁻ is I.

22. Composition comprising a nostocarboline compound according to claim 10 to 21.

23. Composition according to claim 22 comprising further biocidal agents, antifouling agents or herbicides.

24. Polymer matrix, wherein the nostocarboline compound according to claim 10 to 21 is entrapped by extrusion.

25. Polymer emulsion comprising the nostocarboline compound according to claim 10 to 21.

26. Composition comprising a nostocarboline compound according to claim 10 to 21 in the concentration range of 0.1 to 100 µM, preferably 1 to 75 µM, most preferably 30 to 50 µM.
